(19) Europäisches Patentamt
European Patent Office
Office européen des brevets

(11) Numéro de publication: **0 053 539**
**A1**

(12) **DEMANDE DE BREVET EUROPEEN**

(21) Numéro de dépôt: **81401826.3**

(22) Date de dépôt: **19.11.81**

(51) Int. Cl.³: **C 07 D 501/36**, A 61 K 31/545 // C07D253/06, C07C159/00

(30) Priorité: **20.11.80 FR 8024637**

(43) Date de publication de la demande: **09.06.82 Bulletin 82/23**

(84) Etats contractants désignés: **AT BE CH DE FR GB IT LI LU NL SE**

(71) Demandeur: **RHONE-POULENC INDUSTRIES, 22 Avenue Montaigne, F-75008 Paris (FR)**

(72) Inventeur: **Farge, Daniel, 30 rue des Pins Sylvestres, F-94320 Thiais (FR)**
Inventeur: **Le Roy, Pierre, 2 Allée des Cerisiers, F-94320 Thiais (FR)**
Inventeur: **Moutonnier, Claude, 3 rue Auguste Rodin, F-92350 Le Plessis Robinson (FR)**
Inventeur: **Peyronel, Jean-François, 36 parc d'Ardenay, F-91110 Palaiseau (FR)**

(74) Mandataire: **Herson, Jean et al, RHONE-POULENC RECHERCHES Brevets Pharma 25, quai Paul Doumer, F-92408 Courbevoie Cedex (FR)**

(54) **Nouveaux dérivés de la céphalosporine, leur préparation et les compositions pharmaceutiques qui les contiennent.**

(57) Nouveaux dérivés de la céphalosporine de formule générale (I) dans laquelle R est un radical (amino-2 thiazolyl-4)-2 acétyle, (amino-2 thiazolyl-4)-2 méthoxyimino-2 acétyle, (amino-2 thiazolyl-4)-2 carboxyalcoyloxyimino-2 acétyle [dont la partie carboxyalcoyle peut être représentée par la formule générale (II) dans laquelle R′ et R″, qui sont identiques ou différents, représentent des atomes d'hydrogène ou des radicaux alcoyle contenant 1 à 4 atomes de carbone, ou forment ensemble un radical alcoylène contenant 2 ou 3 atomes de carbone], un radical N-(dioxo-2,3 éthyl-4 pipérazinylcarbonyl) Dα-p.hydroxyphénylglycyle ou N-[(hydroxy-4 méthyl-6 pyridyl-3) carbonyl] Dα-p.hydroxyphénylglycyle, et Het est un radical dioxo-5,6 triazine-1,2,4 yle-3 diversement substitué, ainsi que leurs sels, leur préparation et les compositions pharmaceutiques qui les contiennent.

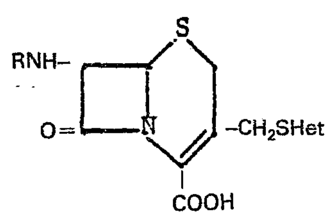
(I)

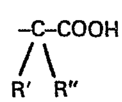
(II)

EP 0 053 539 A1

La présente invention concerne de nouvelles triazinylthiométhylcéphalosporines de formule générale :

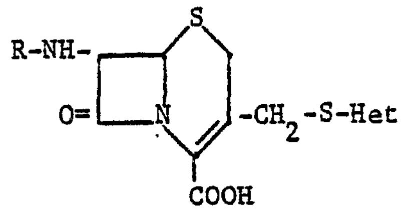

(I)

leurs sels, leur préparation et les médicaments qui les contiennent.

Dans la formule générale (I),

- le symbole R représente un radical (amino-2 thiazolyl-4)-2 acétyle, (amino-2 thiazolyl-4)-2 méthoxyimino-2 acétyle, (amino-2 thiazolyl-4)-2 carboxyalcoyloxyimino-2 acétyle [dont la partie carboxyalcoyle peut être représentée par le radical de formule générale :

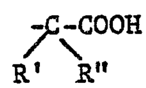

(II)

dans laquelle R' et R" qui sont identiques ou différents représentent des atomes d'hydrogène ou des radicaux alcoyle contenant 1 à 4 atomes de carbone, ou forment ensemble un radical alcoylène contenant 2 ou 3 atomes de carbone], un radical N-(dioxo-2,3 éthyl-4 pipérazinylcarbonyl) Dα-p.hydroxyphénylglycyle ou N-[(hydroxy-4 méthyl-6 pyridyl-3)carbonyl] Dα-p.hydroxyphénylglycyle,

- le symbole Het représente

1°) un radical dioxo-5,6 tétrahydro-1,4,5,6 triazine-1,2,4 yle-3 substitué en position -1, ou -4 ou un radical dioxo-5,6 tétrahydro-1,2,5,6 triazine-1,2,4 yle-3 substitué en position -2 par un radical formylalcoyle

2°) un radical alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 subsitué en position -1 par un radical formylalcoyle.

BAD ORIGINAL

Il est entendu que, sauf mention spéciale, les radicaux et portions alcoyles cités ci-dessus contiennent 1 ou 2 atomes de carbone.

Il est entendu que les groupements méthoxyimino ou carboxyalcoyloxyimino qui interviennent dans certains radicaux R peuvent se trouver dans l'une des positions syn ou anti et que ces isomères et leurs mélanges entrent dans le cadre de la présente invention.

De plus, lorsque R' et R" sont différents, il existe un centre d'asymétrie dans le substituant carboxyalcoyloxyimino. Il est entendu que les formes diastéréoisomères des produits de formule générale (I) qui contiennent de tels radicaux, entrent aussi dans le cadre de la présente invention.

Lorsque le radical Het est un radical tétrahydro-1,4,5,6 triazinyle substitué en position -1 ou -4 ou tétrahydro-1,2,5,6 triazinyle substitué en position -2, il peut être représenté par les formes tautomères :

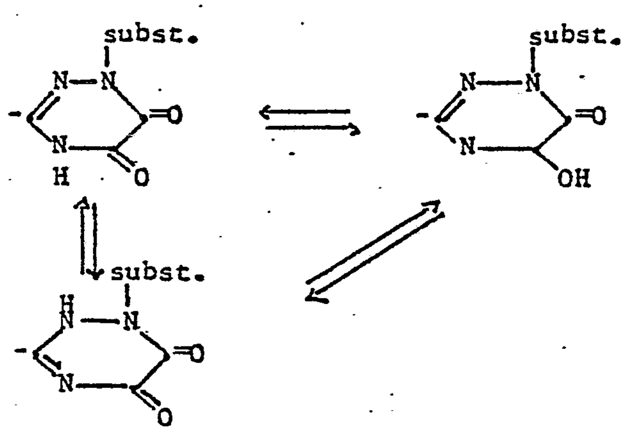

(III a)

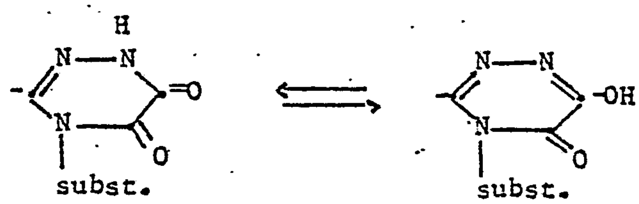

(III b)

ou

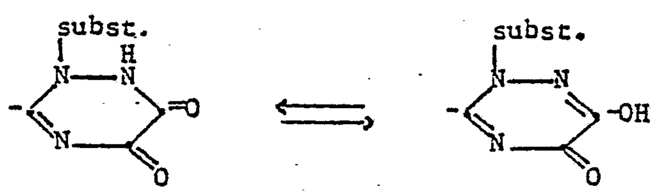

(III c)

Dans le radical Het, le substituant formylalcoyle peut se présenter sous sa forme aldéhyde libre ou hydrate d'aldéhyde. On observe notamment ces formes dans les conditions décrites ci-dessous.

BAD ORIGINAL

Les études de résonance magnétique nucléaire montrent en particulier que lorsque Het est dioxo-5,6 formylméthyl-4 tétrahydro-1,4,5,6 triazine-1,2,4 yle-3 :

- en solvant acide, tel que l'acide formique ou trifluoroacétique (deutérés), en présence ou non d'eau (lourde) le produit se présente principalement sous la forme d'aldéhyde libre.
- en solvant basique tel que l'eau (lourde) additionnée de bicarbonate de sodium, il se présente principalement sous la forme d'hydrate d'aldéhyde.
- en solvant neutre tel que diméthylsulfoxyde ($d_6$), les formes aldéhyde libre et hydrate d'aldéhyde sont présentes, l'addition d'eau déplace l'équilibre en faveur de la forme hydrate d'aldéhyde.

A/ - Selon l'invention les produits de formule générale (I) peuvent être obtenus par acylation d'une amino-7 céphalosporine de formule générale :

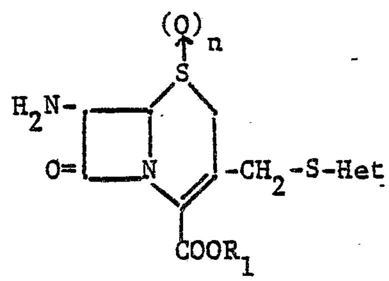

(IV)

dans laquelle Het est défini comme précédemment, $R_1$ est un atome d'hydrogène ou un radical protecteur d'acide facilement éliminable, et n représente 0 ou 1, au moyen d'un acide représenté par la formule générale :

R-OH (V)

dans laquelle R est défini comme précédemment, ou d'un dérivé réactif de cet acide, suivie de la réduction du sulfoxyde lorsque n = 1 et de l'élimination des radicaux protecteurs.

Lorsque $R_1$ est un radical protecteur facilement éliminable, il représente par exemple un radical méthoxyméthyle, t.butyle, benzhydryle, benzyle, nitrobenzyle ou p.méthoxybenzyle.

Il est entendu que lorsque R est un radical (amino-2 thiazolyl-4)-2 méthoxyimino-2 acétyle ou (amino-2 thiazolyl-4)-2 (carboxyalcoyloxyimino-2 acétyle, l'acide de formule générale (V) sous forme syn, anti ou

BAD ORIGINAL

leurs mélanges, conduit respectivement aux produits de formule générale (I) de forme syn, anti ou leurs mélanges.

a) lorsque l'on utilise le produit de formule générale (V) sous forme acide, si R contient un groupement amino, il est nécessaire de protéger ce radical, par exemple par un groupement protecteur d'amine facilement éliminable tel que le groupement t.butoxycarbonyle, trichloro-2,2,2 éthoxycarbonyle, trityle, p.nitrobenzyloxycarbonyle, p.méthoxybenzyloxycarbonyle ou formyle ; lorsque R contient un radical hydroxy, celui-ci peut être libre ou protégé [par exemple par un radical trityle, tétrahydropyrannyle, méthoxy-2 propyle-2, alcoyloxycarbonyle (tel que t.butoxycarbonyle) ou aryloxycarbonyle (tel que benzyloxycarbonyle)] ; lorsque R contient un groupement carboxy, il est aussi nécessaire de protéger ce radical, par exemple par un radical protecteur tel que défini précédemment pour $R_1$.

On condense le produit de formule générale (V) sous sa forme acide sur l'amino-7 céphalosporine de formule générale (IV) dans laquelle $R_1$ représente un radical protecteur facilement éliminable, en opérant dans solvant organique tel que le diméthylformamide, l'acétonitrile, le tétrahydrofuranne, le chlorure de méthylène ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre -20 et 40°C, puis on réduit l'oxyde obtenu (lorsque l'on a utilisé une amino-7 céphalosporine de formule générale (IV) dans laquelle n = 1), et on élimine les groupements protecteurs. Eventuellement on opère en présence d'une quantité catalytique de NN-diméthylamino-4 pyridine.

Il est entendu que le radical formylalcoyle contenu dans le radical Het peut être éventuellement protégé à l'état d'acétal, sous forme d'un radical de formule générale :

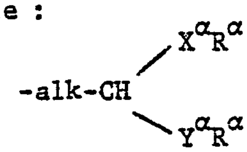

(VI)

dans laquelle alk est un radical alcoylène contenant 1 ou 2 atomes de

carbone, $X^{\alpha}$ et $Y^{\alpha}$ sont identiques et représentent des atomes d'oxygène ou de soufre, et $R^{\alpha}$ représente un radical alcoyle, ou bien $X^{\alpha}$ et $Y^{\alpha}$ sont identiques ou différents et représentent des atomes d'oxygène ou de soufre, et les radicaux $R^{\alpha}$ forment ensemble un radical alcoylène contenant 2 ou 3 atomes de carbone.

La libération du radical formylalcoyle est effectuée après la réduction de l'oxyde, avant, simultanément ou après l'élimination des autres radicaux protecteurs.

La réduction du S-oxyde s'effectue par exemple dans les conditions décrites dans la demande de brevet allemand 2 637 176.

L'élimination des différents radicaux protecteurs peut s'effectuer simultanément ou successivement.

A titre d'exemple,

1/ L'élimination des groupements protecteurs d'amines s'effectue

- lorsqu'il s'agit d'un radical t.butoxycarbonyle, trityle, p.méthoxybenzyloxycarbonyle ou formyle : par traitement en milieu acide. De préférence on utilise l'acide trifluoracétique en opérant à une température comprise entre 0 et 20°C, ou bien on utilise l'acide formique, phosphorique ou polyphosphorique purs ou en présence d'eau, à température comprise entre 20 et 60°C, ou encore l'acide paratoluènesulfonique ou méthanesulfonique dans l'acétone ou l'acétonitrile, ou l'acide chlorhydrique anhydre, à une température comprise entre 20°C et la température de reflux du mélange réactionnel. Dans ces conditions le produit de formule générale (I) peut être obtenu sous forme de trifluoracétate, de solvate avec l'acide formique, de phosphate, de méthanesulfonate, de paratoluènesulfonate ou de chlorhydrate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique ;

- lorsqu'il s'agit d'un radical trichloro-2,2,2 éthoxycarbonyle ou p.nitrobenzyloxycarbonyle : par réduction (notamment traitement par le zinc dans l'acide acétique) ;

2/ L'élimination des groupements protecteurs du radical carboxy s'effectue :

- lorsqu'il s'agit d'un groupement t.butyle, p.méthoxybenzyle ou benzhydryle : par traitement en milieu acide, dans les conditions décrites ci-dessus pour l'élimination du radical trityle protecteur d'amino. Dans le cas du radical benzhydryle, on peut opérer en présence d'anisole ;
- lorsqu'il s'agit d'un groupement méthoxyméthyle : par traitement en milieu acide dilué ;
- lorsqu'il s'agit d'un groupement nitrobenzyle : par réduction (notamment traitement par le zinc dans l'acide acétique ou hydrogénolyse).
- lorsqu'il s'agit du groupement méthoxy-2 propyle-2 : selon la méthode décrite dans le brevet belge 875 379.

3/ Le déblocage des groupements de formule générale (VI) (pour obtenir un produit de formule générale (I) dans laquelle Het contient un radical formylalcoyle), s'effectue :

- en présence d'un acide sulfonique (par exemple acide méthanesulfonique ou acide p.toluènesulfonique) dans un solvant organique (par exemple acétonitrile ou acétone), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel ;
- ou bien, par action d'acide formique pur ou aqueux (contenant de préférence moins de 10 % d'eau), soit en présence ou non de silice, soit par transacétalisation en présence d'un réactif acétalisable tel que défini ci-dessus.

4/ L'élimination des groupements protecteurs d'hydroxy s'effectue :

- lorsqu'il s'agit d'un groupement trityle ou tétrahydropyrannyle : par acidolyse, par exemple par l'acide trifluoracétique, l'acide formique aqueux ou non ou l'acide paratoluènesulfonique ;
- lorqu'il s'agit du groupement méthoxy-2 propyle-2 : selon la méthode décrite dans la brevet belge 875 379 ;
- lorsqu'il s'agit des groupements alcoyloxycarbonyle ou aryloxycarbonyle : selon les méthodes décrites dans le brevet belge 871 213.

b) Lorsque l'on utilise le produit de formule générale (V) sous forme d'un dérivé réactif, on met en oeuvre par exemple l'anhydride, un anhydride mixte ou un ester réactif représenté par la formule générale :

$$R\text{-}OZ \qquad (VII)$$

dans laquelle Z représente un radical succinimido, benzotriazolyle-1, nitro-4 phényle, dinitro-2,4 phényle, pentachlorophényle ou phtalimido. Le cas échéant la fonction amine de ces dérivés est préalablement protégée. Il en est de même lorsque ces dérivés contiennent un radical acide. Lorsque le radical R contient un substituant hydroxy, celui-ci est libre ou protégé.

Il est également possible de mettre en oeuvre un dérivé réactif tel qu'un halogénure d'acide. Dans ce dernier cas, lorsque R contient un radical amino, on peut faire réagir le chlorhydrate du chlorure d'acide (étant entendu que lorsque ces dérivés contiennent une autre fonction acide, il est nécessaire que celle ci soit protégée).

La condensation de l'anhydride, de l'anhydride mixte, ou de l'halogénure d'acide (qui peuvent être préparés in situ) s'effectue sur l'amino-7 céphalosporine de formule générale (IV) dans laquelle $R_1$ est un atome d'hydrogène ou un radical protecteur facilement éliminable, en opérant dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone), ou dans des mélanges de tels solvants en présence d'un accepteur d'acide tel qu'un époxyde (par exemple l'oxyde de propylène) ou une base organique azotée comme la pyridine, la diméthylaminopyridine, la N-méthylmorpholine ou une trialcoylamine (par exemple triéthylamine), ou dans un milieu hydroorganique en présence d'un agent alcalin de condensation tel que le bicarbonate de sodium, et l'on opère à une température comprise entre -40 et +40°C, puis on réduit, le cas échéant, le S-oxyde obtenu, et on remplace éventuellement les groupements protecteurs par des atomes d'hydrogène.

Lorsque l'on met en oeuvre un ester réactif de formule générale (VII), la réaction s'effectue dans les conditions décrites ci-dessus, en opérant éventuellement en présence d'une trialcoylamine (par exemple triéthylamine) dans un solvant organique tel que diméthylformamide à une température comprise entre 0 et 40°C, puis on réduit, le cas échéant, le S-oxyde obtenu et on remplace les groupements protecteurs par des atomes d'hydrogène.

B-/ Selon l'invention les produits de formule générale (I) peuvent également être préparés par action d'un thiol de formule générale :

Het-SH (VIII)

(ou d'un de ses sels alcalins ou alcalino-terreux), [dans laquelle Het, qui est défini comme ci-dessus, est protégé à l'état d'acétal tel que défini par la formule générale (VI)] sur un dérivé de céphalosporine de formule générale :

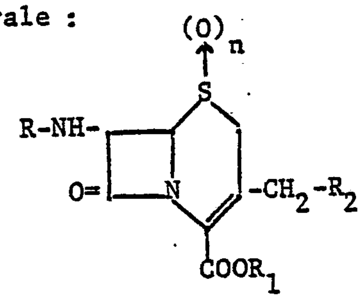

(IX)

[dans laquelle, R étant défini comme précédemment, $R_2$ est un atome d'halogène choisi parmi le chlore, le brome et l'iode, $R_1$ est défini comme précédemment et n est égal à 0 ou 1, ou bien $R_2$ est un radical acétoxy, $R_1$ est un atome d'hydrogène et n est égal à 0], suivie de la réduction de l'oxyde obtenu lorsque n = 1 puis de l'élimination des radicaux protecteurs.

Il est entendu que les radicaux amino ou hydroxy contenus dans R peuvent être libres ou protégés.

On opère notamment dans un solvant organique, par exemple le diméthylformamide ou l'acétonitrile, ou un mélange de tels solvants à une température comprise entre 20°C et la température de reflux du mélange réactionnel.

Lorsque l'on met en oeuvre une céphalosporine de formule générale (IX) dans laquelle $R_2$ est un radical acétoxy, on opère de préférence à une température supérieure à 50°C si l'on effectue la réaction en milieu organique ; on peut aussi opérer en milieu aqueux en présence d'un bicarbonate alcalin ou en milieu hydroorganique (par exemple acétone-eau, diméthylformamide-eau, acétonitrile-eau) à une température comprise entre 20 et 80°C.

Lorsque l'on met en oeuvre une céphalosporine de formule générale (IX) dans laquelle $R_2$ représente un atome d'halogène, on opère de préférence en présence d'une base organique telle qu'une pyridine ou une base organique tertiaire du type :

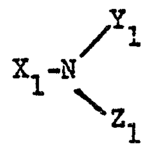

(X)

ou $X_1$, $Y_1$ et $Z_1$ représentent des radicaux alcoyle ou phényle, ou éventuellement deux d'entre eux forment un cycle avec l'atome d'azote auquel ils sont rattachés. On utilise par exemple la diisopropyléthylamine ou la diéthylphénylamine. La présence d'une base organique n'est pas nécessaire si l'on fait agir un sel alcalin ou alcalino-terreux du thiol de formule générale (VIII). Outre les solvants déjà cités ci-dessus on peut également utiliser avantageusement le tétrahydrofuranne ou le diméthylacétamide. Il est aussi possible d'opérer en présence d'eau. La réaction peut aussi s'effectuer à une température plus basse allant jusqu'à -20°C.

Eventuellement on opère sous azote.

La réduction de l'oxyde et l'élimination des groupements protecteurs s'effectuent selon les méthodes décrites précédemment.

C-/ Selon l'invention, les produits de formule générale (I) peuvent également être préparés par action d'un thioloester de formule générale :

R-S Het (XI)

dans laquelle R et Het sont définis comme précédemment [étant entendu que lorsque R contient un substituant amino ou carboxy, celui-ci est protégé, et lorsqu'il contient un substituant hydroxy, celui-ci est libre ou protégé et le substituant formylalcoyle de Het est protégé à l'état d'acétal de formule générale (VI)] sur une amino-7 céphalosporine de formule générale :

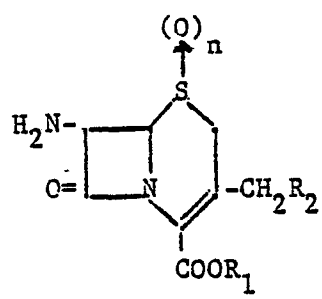

(XII)

dans laquelle $R_1$, $R_2$ et n sont définis comme précédemment pour la formule générale (IX), suivie de la réduction du sulfoxyde lorsque n = 1 et de l'élimination des radicaux protecteurs.

De même que pour le procédé A-/, lorsque R est un radical (amino-2 thiazolyl-4)-2 méthoxyimino-2 acétyle ou (amino-2 thiazolyl-4)-2 carboxyalcoyloxyimino-2 acétyle, il est entendu que les thioloesters de forme syn, anti, ou leurs mélanges conduisent respectivement aux produits de formule générale (I) de forme syn, anti ou leurs mélanges.

La réaction s'effectue avantageusement dans un solvant organique tel qu'un amide (par exemple diméthylformamide, diméthylacétamide), un éther (par exemple tétrahydrofuranne, dioxanne), un solvant chloré (par exemple chloroforme, chlorure de méthylène), une cétone (par exemple acétone) ou un nitrile (par exemple acétonitrile), ou bien dans un mélange de tels solvants. Il est également possible d'opérer en présence d'une base minérale dans l'un des solvants cités ci-dessus, éventuellement en présence d'eau, à un pH compris entre 6,7 et 7,5.

Lorsque l'on met en oeuvre une amino-7 céphalosporine de formule générale (XII) dans laquelle $R_2$ est un radical acétoxy, on opère de préférence en milieu hydroorganique, ou même en milieu aqueux en présence d'une base minérale citée ci-dessus. La réaction s'effectue entre 0°C et la température de reflux du mélange réactionnel.

Lorsque l'on met en oeuvre un produit de formule générale (XII) dans laquelle $R_2$ est un atome d'halogène, on opère généralement en présence d'un accepteur d'acide tel qu'une base organique par exemple telle que citée précédemment en B-/, notamment la pyridine, la triéthylamine, la diisopropyléthylamine, la diéthylphénylamine ou la N-méthylmorpholine. La réaction s'effectue de préférence en milieu anhydre, à une température comprise entre -20°C et la température de reflux du mélange réactionnel. On opère éventuellement sous azote.

La réduction du sulfoxyde, la protection et l'élimination des radicaux protecteurs s'effectuent dans les conditions décrites précédemment.

D-/ Selon l'invention, les produits de formule générale (I) dans laquelle R est un radical (amino-2 thiazolyl-4)-2 acétyle, (amino-2 thiazolyl-4)-2

méthoxyimino-2 acétyle ou (amino-2 thiazolyl-4)-2 carboxyalcoyloxyimino-2 acétyle peuvent également être préparés par action d'une thiourée de formule générale :

$$R_4NHCSNH_2 \qquad (XIII)$$

dans laquelle $R_4$ est un atome d'hydrogène ou un radical tel que défini précédemment pour les radicaux protecteurs d'amino, sur un produit de formule générale :

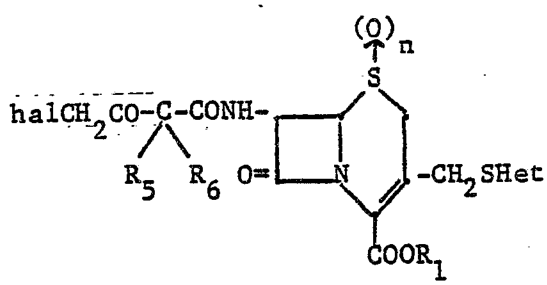

(XIV)

[dans laquelle Het, $R_1$ et n sont définis comme précédemment, hal représente un atome de chlore ou de brome, et $R_5$ et $R_6$ soit représentent simultanément des atomes d'hydrogène, soit forment ensemble un radical méthoxyimino ou carboxyalcoyloxyimino tel que défini précédemment (étant entendu que ce dernier radical peut être libre ou protégé)], suivie de la réduction du sulfoxyde lorsque n = 1 et éventuellement de l'élimination des radicaux protecteurs.

On opère généralement en milieu aqueux, organique ou hydroorganique par exemple dans des solvants ou des mélanges de solvants tels que des alcools (méthanol, éthanol), des cétones (acétone), des solvants chlorés (chloroforme, chlorure d'éthylène), des nitriles (acétonitrile), des amides (diméthylformamide, diméthylacétamide), des éthers (tétrahydrofuranne, dioxanne), des esters (acétate d'éthyle) ou des acides (acide acétique, acide formique), en présence ou non d'une base telle que la soude, la potasse, les carbonates, les carbonates acides des métaux alcalins, les sels d'acides carboxyliques et de métaux alcalins (formiate de sodium, acétate de sodium) ou les amines tertiaires (triéthylamine, triméthylamine ou pyridine), à une température comprise entre -30 et 60°C.

Lorsque l'on opère en présence d'une base, selon la nature de celle-ci et la quantité introduite, on isole ou non l'intermédiaire de formule générale :

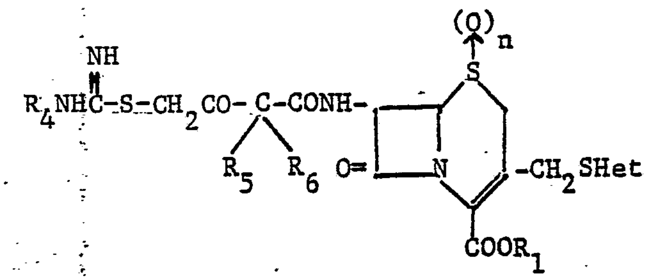

(XIV a)

dans laquelle $R_1$, $R_4$, $R_5$, $R_6$, Het et n sont définis comme précédemment, qui peut alors être cyclisé en milieu acide.

Le radical Het peut contenir le substituant formylalcoyle à l'état libre ou protégé sous forme d'acétal tel que défini précédemment.

La réduction du sulfoxyde et l'élimination le cas échéant des radicaux protecteurs s'effectuent dans les conditions décrites précédemment.

Les produits de formule générale (IV) peuvent être obtenus selon l'une ou l'autre des méthodes suivantes :

I-/ A partir d'une céphalosporine de formule générale :

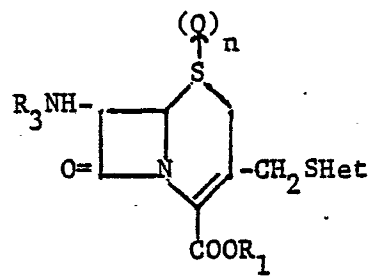

(XV)

[dans laquelle $R_1$, Het et n sont définis comme précédemment et $R_3$ est un radical facilement éliminable], par élimination du radical $R_3$, ou éventuellement élimination du radical $R_3$ et simultanément des autres radicaux protecteurs puis, éventuellement, réduction du sulfoxyde obtenu lorsque n = 1 et élimination des autres radicaux protecteurs.

Par radical facilement éliminable on entend l'un des groupements protecteurs d'amino par exemple tels que définis précédemment en A-/ pour la protection du groupement amino de R.

L'élimination s'effectue lorsqu'il s'agit des radicaux t.butoxycarbonyle, trityle, p.méthoxybenzyloxycarbonyle ou formyle :

- lorsque l'on veut éliminer uniquement le radical $R_3$ : par traitement dans le méthanol, par une solution d'acide chlorhydrique anhydre dans un éther,

-lorsque l'on veut éliminer simultanément le radical $R_3$ et le radical $R_1$ (lorsque ce dernier représente un radical benzhydryle, t.butyle ou p.méthoxybenzyle), par traitement par l'acide chlorhydrique concentré dans le méthanol selon le brevet belge 872 616 ;

-lorsque l'on veut éliminer les radicaux $R_3$ et $R_1$ ci-dessus et le groupement protecteur du substituant formylalcoyle du radical Het : par traitement par l'acide formique pur ou par un acide sulfonique, puis par traitement soit par une solution d'acide chlorhydrique anhydre dans un éther (par exemple dioxanne) soit par une solution d'acide chlorhydrique concentré dans un éther.

Les céphalosporines de formule générale (XV) peuvent être obtenues par action d'un thiol de formule générale (VIII) ou d'un de ses sels alcalins ou alcalino-terreux, sur une céphalosporine de formule générale :

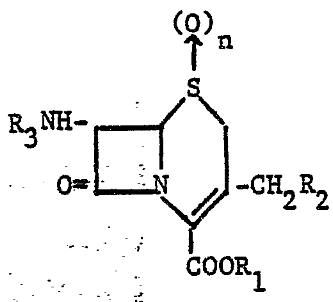

(XVI)

dans laquelle $R_3$ est défini comme précédemment et $R_1$, $R_2$ et n sont définis comme précédemment pour la formule générale (IX), puis éventuellement réduction du sulfoxyde obtenu lorsque n = 1 et élimination des radicaux protecteurs.

On opère dans les conditions décrites précédemment pour faire réagir un thiol de formule générale (VIII) avec une céphalosporine de formule générale (IX).

Les produits de formule générale (XVI) dans laquelle $R_3$, $R_1$ et n sont définis comme ci-dessus et $R_2$ est un atome d'halogène, peuvent être préparés selon la méthode décrite dans le brevet américain 4 042 585.

Les produits de formule générale (XVI) dans laquelle $R_3$, $R_1$ et n sont définis comme ci-dessus et $R_2$ est un radical acétoxy peuvent être obtenus à partir de l'amino-7 céphalosporine correspondante par application des méthodes connues ou décrites dans la littérature.

- lorsque $R_3$ est un radical formyle : selon J.C. SHEEHAN et coll., J. Amer. Chem. Soc. 80 1156 (1958),
- lorsque $R_3$ est un radical t.butoxycarbonyle: selon L. MORODER et coll., Hoppe Seyler's Z. Physiol. Chem. 357 1651 (1976),
- lorsque $R_3$ est trichloro-2,2,2 éthoxycarbonyle, p.nitrobenzyloxycarbonyle ou p.méthoxybenzyloxycarbonyle : par action d'un chloroformiate en milieu hydroorganique en présence d'un bicarbonate alcalin,
- lorsque $R_3$ est trityle: selon la méthode décrite dans le brevet américain 4 039 534,
- lorsque $R_1$ est méthoxyméthyle: selon S. SEKI et coll., Tetrahedron Lett., 33, 2915 (1977),
- lorsque $R_1$ est t.butyle: selon R.J. STEDMAN, J. Med. Chem., 9, 444 (1966),
- lorsque $R_1$ est benzhydryle: selon la demande de brevet néerlandais 73 03263,
- lorsque $R_1$ est nitrobenzyle ou p.méthoxybenzyle: selon R.R. CHAUVETTE et coll., J. Org. Chem. 38 (17), 2992 (1973).

II/ Les produits de formule générale (IV) peuvent aussi être préparés par action d'un thiol de formule générale (VIII) (ou d'un de ses sels alcalins ou alcalino-terreux) sur une amino-7 céphalosporine de formule générale (XII) dans laquelle $R_1$, $R_2$ et n sont définis comme précédemment, suivie éventuellement de la réduction du sulfoxyde obtenu lorsque n = 1 et de l'élimination des radicaux protecteurs.

La réaction s'effectue dans les conditions décrites précédemment pour l'action de (VIII) sur (IX).

Les produits de formule générale (XII) dans laquelle $R_1$ et n sont définis comme précédemment et $R_2$ est un atome d'halogène peuvent être obtenus selon la méthode décrite dans le brevet américain 4 042 585.

L'acide de formule générale (V) dans laquelle R est un radical (amino-2 thiazolyl-4)-2 acétyle peut être préparé selon la méthode décrite dans la demande de brevet allemand 2 534 850.

L'acide de formule générale (V) dans laquelle R est un radical (amino-2 thiazolyl-4)-2 méthoxyimino-2 acétyle peut être préparé selon la méthode décrite dans le brevet belge 872 616.

Les acides de formule générale (V) dans laquelle R est un radical (amino-2 thiazolyl-4)-2 carboxyalcoyloxyimino-2 acétyle peuvent être obtenus par application des méthodes décrites dans les brevets belges 864 810, 865 298, 876 541 et 876 542.

L'acide de formule générale (V) dans laquelle R est un radical N-(dioxo-2,3 éthyl-4 pipérazinylcarbonyl) Dα-p.hydroxyphénylglycyle peut être préparé selon la méthode décrite dans le brevet belge 837 682.

L'acide de formule générale (V) dans laquelle R est un radical N-[(hydroxy-4 méthyl-6 pyridyl-3)carbonyl] D-α-p.hydroxyphénylglycyle peut être préparé selon la méthode décrite dans les demandes de brevet japonais publiées sous les numéros 52 156 888, 52 156 889 et 52 156 890.

Les thiols de formule générale (VIII)(qui peuvent être mis en oeuvre sous leur forme tautomère), peuvent être préparés par application des méthodes suivantes selon la signification du radical Het :

- lorsque Het est un radical défini dans la formule générale (I) en 1°/ (protégé à l'état d'acétal), on fait agir un oxalate d'alcoyle ou un halogénure de mono oxalate d'alcoyle sur une thiosemicarbazide de formule générale :

$H_2NCSNHNH-R^{\gamma}$

(XVII a)

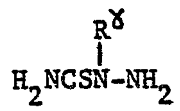

(XVII b)

$R^{\gamma}NHCSNHNH_2$

(XVII c)

dans laquelle $R^{\gamma}$ est un radical de formule générale (VI) en présence d'un alcoolate alcalin, par exemple l'éthylate ou le méthylate de sodium ou le t.butylate de potassium, par application de la méthode décrite par M. PESSON et M. ANTOINE, Bull. Soc. Chim. France 1590 (1970).

Il n'est pas absolument nécessaire de purifier le produit obtenu (ni de libérer les radicaux protégés) pour le mettre en oeuvre pour la préparation des produits de formule générale (I).

Les thiosemicarbazides de formules générales (XVIIa), (XVIIb) et (XVIIc) peuvent être préparées selon l'une des méthodes décrites par K.A. JENSEN et coll., Acta Chem. Scand., 22, 1 (1968), ou par application de la méthode décrite par Y. KAZAKOV et J.Y. POTOVSKII, Doklady Acad. Nauk. SSSR 134, 824 (1960).

- Lorsque Het est un radical défini dans la formule générale (I) en 2°/ (protégé à l'état d'acétal) on fait agir un oxalate d'alcoyle sur une thiosemicarbazide, par analogie avec la méthode décrite par M. PESSON et M. ANTOINE, C.R. Acad. Sci. 267, 15C, 904 (1968) ou C.R. Acad. Sci. 267, 25, 1726 (1968).

Les céphalosporines de formule générale (IX) peuvent être préparées par acylation d'une amino-7 céphalosporine de formule générale (XII) dans laquelle $R_1$, $R_2$ et n sont définis comme précédemment, suivie éventuellement de la réduction du sulfoxyde lorsque n = 1 et de l'élimination des radicaux protecteurs (notamment de l'élimination du radical protecteur $R_1$ lorsque $R_2$ est acétoxy).

L'acylation s'effectue au moyen d'un acide de formule générale (V) ou d'un de ses dérivés réactifs, dans les conditions décrites précédemment pour l'obtention des céphalosporines de formule générale (I) à partir des produits (IV) et (V).

Les thioloesters de formule générale (XI) peuvent être préparés par action d'un acide de formule générale (V) ou d'un de ses dérivés réactifs (la fonction amine qui peut être contenue par R étant obligatoirement protégée excepté lorsque l'on utilise le chlorhydrate du chlorure d'acide) sur un thiol de formule générale (VIII) ou sur un sel alcalin ou alcalino-terreux de ce thiol.

Il est entendu que lorsque R contient un radical carboxy, celui-ci est protégé préalablement, et lorsque R contient un radical hydroxy, ce dernier est libre ou protégé.

a/ Lorsque l'on utilise le produit de formule générale (V) sous forme acide, la condensation s'effectue généralement dans un solvant organique tel que le diméthylformamide, l'acétonitrile, le tétrahydrofuranne, le chlorure de méthylène, le chloroforme ou l'acétate d'éthyle, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre -20 et 40°C.

b/ Lorsque l'on utilise un dérivé réactif de l'acide de formule générale (V), il est possible de mettre en oeuvre l'anhydride, un anhydride mixte, un halogénure d'acide ou un ester réactif de formule générale (VII).

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone), ainsi que des mélanges de tels solvants, en présence d'un accepteur d'acide tel qu'un époxyde (par exemple oxyde de propylène) ou tel qu'une base organique azotée comme la pyridine, la N-méthylmorpholine ou une trialcoylamine (par exemple triéthylamine) ou dans un milieu hydroorganique en présence d'un agent alcalin de condensation tel que le bicarbonate de sodium, et l'on opère à une température comprise entre -40 et +40°C, puis on élimine éventuellement le ou les groupements protecteurs.

Lorsque l'on met en oeuvre un ester réactif de formule générale (VII), on opère généralement en présence d'une trialcoylamine (par exemple triéthylamine) dans un solvant organique tel que le diméthylformamide à une température comprise entre 0 et 60°C.

Les céphalosporines de formule générale (XIV) peuvent être obtenues par action d'un halogénure d'acide de formule générale :

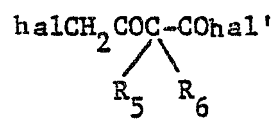

(XVIII)

dans laquelle hal et hal' sont des atomes de chlore ou de brome et $R_5$ et $R_6$ sont définis comme précédemment (à l'exception de représenter un radical carboxyalcoyloxyimino libre), sur une amino-7 céphalosporine de formule générale (IV), suivie éventuellement de la réduction du sulfoxyde obtenu lorsque n = 1 et de l'élimination des radicaux protecteurs.

Il est entendu que le radical formylalcoyle contenu par Het est libre ou protégé.

La réaction s'effectue généralement en milieu hydroorganique par exemple eau-éther (tétrahydrofuranne, dioxanne), eau-cétone (acétone) ou eau-solvant chloré (chloroforme, chlorure de méthylène), en présence d'un agent alcalin de condensation tel qu'un bicarbonate alcalin (par exemple bicarbonate de sodium) à une température comprise entre -40 et +40°C.

Il est également possible d'opérer par analogie avec la méthode décrite dans la demande de brevet français 2 399 418.

La réduction du sulfoxyde et l'élimination des radicaux protecteurs s'effectuent dans les conditions décrites précédemment.

Les produits de formule générale (XVIII) peuvent être obtenus par halogénation d'un produit de formule générale :

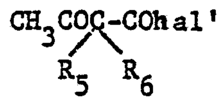

(XIX)

[dans laquelle $R_5$, $R_6$ et hal' sont définis comme précédemment dans la formule générale (XVIII)], par toute méthode connue en soi pour la préparation des dérivés halogénés, qui n'altère pas le reste de la molécule.

Lorsque l'on veut obtenir un produit de formule générale (XVIII) dans laquelle hal représente un atome de brome, on fait agir le brome en présence d'un catalyseur, soit un catalyseur acide tel que l'acide bromhydrique, l'acide chlorhydrique, les acides sulfoniques (acide méthanesulfonique, acide p.toluènesulfonique anhydre ou acide benzènesulfonique), soit en présence de lumière ultra-violette.

Lorsque l'on veut obtenir un produit de formule générale (XVIII) dans laquelle hal est un atome de chlore, on fait agir le chlore en présence d'un catalyseur tel que cité ci-dessus ou le chlorure de sulfuryle.

L'halogénation s'effectue dans un solvant organique tel qu'un solvant chloré (par exemple chlorure de méthylène, chloroforme, tétrachlorure de carbone, dichloroéthane ou trichloroéthane) ou un éther (par exemple éther éthylique ou dioxanne) ou dans un mélange de tels solvants, à une température comprise entre -40°C et la température de reflux du mélange réactionnel.

Les produits de formule générale (XIX) pour lesquels $R_5$ et $R_6$ représentent des atomes d'hydrogène sont préparés selon la méthode décrite dans la demande de brevet français 2 399 418.

Les produits de formule générale (XIX) peuvent être préparés par action d'un agent d'halogénation sur l'acide, un sel ou un ester silylique de l'acide de formule générale :

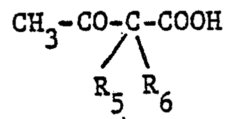 (XX)

(dans laquelle $R_5$ et $R_6$ sont des radicaux méthoxyimino ou carboxyalcoyloxyimino protégé) en présence d'un produit de formule générale :

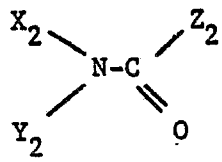 (XXI)

dans laquelle $X_2$, $Y_2$ et $Z_2$ sont identiques ou différents et représentent des radicaux alcoyle ou phényle, ou deux d'entre eux forment ensemble avec les atomes auxquels ils sont rattachés un hétérocycle à 5 ou 6 chaînons contenant éventuellement un autre hétéroatome choisi parmi l'oxygène, l'azote ou le soufre, ou bien $X_2$ et $Y_2$ représentent des radicaux alcoyle tels que définis précédemment et $Z_2$ représente un atome d'hydrogène ou un radical dialcoylamino.

L'agent d'halogénation peut être choisi parmi le pentachlorure de phosphore, l'oxychlorure de phosphore, le chlorure d'oxalyle ou le phosgène lorsque l'on veut obtenir le chlorure d'acide, ou bien parmi le pentabromure de phosphore, l'oxybromure de phosphore ou le bromure de thionyle lorsque l'on veut obtenir le bromure d'acide.

Parmi les produits de formule générale (XXI) on utilise avantageusement le diméthylacétamide, le diéthylacétamide, le diméthylpropionamide, le diéthylpropionamide, le diméthylformamide, la N-acétylmorpholine, la N-acétylpipéridine, la N-acétyl N-méthylaniline, la N-méthylpyrrolidone ou la tétraméthylurée.

Lorsque l'on fait agir un sel de l'acide de formule générale (XX), on met en oeuvre de préférence un sel alcalin ou un sel d'amine tertiaire (par exemple de triéthylamine).

Lorsque l'on fait agir un ester silylique, on met en oeuvre avantageusement un ester de triméthylsilyle ou de t.butyldiméthylsilyle. Cet ester peut éventuellement être préparé par application de la méthode décrite par A. WISSNER et coll., J. Org. Chem., 43 (20), 3972 (1978), ou généré in situ.

L'halogénation de l'acide de formule générale (XX) s'effectue généralement dans un solvant organique tel qu'un solvant chloré (par exemple dichlorométhane, chloroforme), dans un ester (par exemple acétate d'éthyle), un hydrocarbure aromatique (par exemple toluène, xylène), ou dans un éther (par exemple éther éthylique, éther isopropylique ou tétrahydrofuranne), à une température comprise entre -70 et +30°C.

Il est entendu que les acides de formule générale (XX) de forme syn conduisent aux halogénures d'acide de forme syn et que les acides de formule générale (XX) de forme anti conduisent aux halogénures d'acide de formule générale (XIX) de forme anti.

Les acides de formule générale (XX) peuvent être obtenus par hydrolyse acide ou par saponification d'un ester de formule générale :

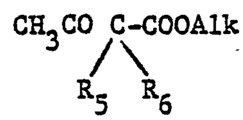

(XXII)

dans laquelle $R_5$ et $R_6$ sont définis comme ci-dessus pour la formule générale (XX) et Alk représente un radical alcoyle.

On opère généralement en présence de soude dans l'éthanol, à la température de reflux du mélange réactionnel.

Les esters de formule générale (XXII) peuvent être préparés par application de la méthode décrite par R. BUCOURT et coll., Tetrahedron 34, 2233 (1978).

Les acides de formule générale (XX) dans laquelle $R_5$ et $R_6$ représentent un radical carboxyalcoyloxyimino protégé peuvent être préparés par action d'un produit de formule générale :

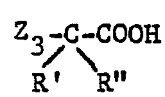

(XXIII)

(dans laquelle R' et R" sont définis comme précédemment et $Z_3$ est un atome d'halogène ou un radical sulfate ou sulfonate, et dont la fonction acide est protégée) sur un produit de formule générale :

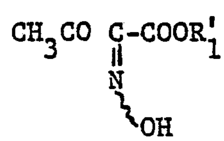

(XXIV)

dans laquelle $R'_1$ est un radical protecteur d'acide tel que défini précédemment pour $R_1$, puis élimination du radical protecteur $R'_1$.

La réaction s'effectue généralement en présence d'une base minérale ou organique (par exemple la soude, la potasse, l'hydrure de sodium, le carbonate de potassium ou une base azotée telle que la triéthylamine) dans un solvant organique tel qu'un solvant chloré (par exemple dichlorométhane, dichloroéthane), un éther (par exemple tétrahydrofuranne, dioxanne), une cétone (par exemple acétone) ou un amide (par exemple diméthylformamide).

Il est nécessaire que le radical protecteur d'acide $R'_1$ et le radical protecteur de la fonction acide du produit de formule générale (XXIII) soient différents et éliminables sélectivement.

L'élimination du radical protecteur $R'_1$ s'effectue dans les conditions décrites précédemment.

Les produits selon la présente invention peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration ou décantation. Il peut également être isolé de sa solution par lyophilisation.

Les nouveaux produits selon l'invention pour lesquels R contient un radical amino peuvent aussi être transformés en sels d'addition avec les acides. Selon les procédés de la présente invention les produits peuvent être obtenus sous forme de trifluoroacétate, solvate avec l'acide formique ou l'eau, phosphate, paratoluènesulfonate, méthanesulfonate ou chlorhydrate. Les produits de formule générale (I), obtenus sous forme de ces sels, peuvent être libérés et transformés en sels d'autres acides selon les méthodes habituelles.

Comme exemples de sels pharmaceutiquement acceptables peuvent être cités les sels avec les métaux alcalins (sodium, potassium, lithium), avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyl-éthanolamine, benzylamine, dicyclohexylamine, N-benzyl-β-phénéthylamine, NN'-dibenzyléthylènediamine, diphénylènediamine, benzhydrylamine, quinine, chloline, arginine, lysine, leucine, dibenzylamine) ou les sels d'addition avec des acides minéraux (chlorhydrates, bromhydrates, sulfates, nitrates, phosphates) ou organiques (succinates, fumarates, maléates, p.toluène-sulfonates).

Les nouveaux produits selon la présente invention peuvent être éventuellement purifiés par des méthodes physiques telles que la cristallisation ou la chromatographie.

Les nouveaux dérivés de la céphalosporine selon la présente invention et leurs sels pharmaceutiquement acceptables présentent des propriétés anti-bactériennes particulièrement intéressantes. Ils manifestent une activité remarquable in vitro et in vivo sur les germes Gram-positifs et Gram-négatifs.

In vitro, les produits de formule générale (I) se sont montrés actifs à une concentration comprise entre 5 et 15 μg/cm3 sur des souches de staphylocoques sensibles à la pénicilline G (Staphylocoque aureus Smith), à une concentration comprise entre 0,01 et 2 μg/cm3 sur Escherichia coli souche NIHJ.

In vivo les produits de formule générale (I) se sont montrés actifs sur les infections expérimentales de la souris à Staphylococcus aureus Smith (sensible à la pénicilline G) à une dose comprise entre 1 et 15 mg/kg par jour par voie sous-cutanée, et à Escherichia coli (souche Monod) à des doses comprises entre 0,01 et 10 mg/kg par jour par voie sous-cutanée.

Par ailleurs, la $DL_{50}$ des produits de formule générale (I) est comprise entre 1 g/kg et des doses supérieures à 2,5 g/kg par voie sous-cutanée chez la souris.

D'un intérêt particulier sont les produits de formule générale (I) dans laquelle Het représente un radical dioxo-5,6 formylalcoyle-4 tétrahydro-1,4,5,6 triazine-1,2,4 yle-3.

L'exemple suivant, donné à titre non limitatif, illustre la présente invention.

EXEMPLE

On agite à 20°C pendant 10 minutes une suspension de 2,99 g de chlorhydrate d'amino-7 benzhydryloxycarbonyl-2 [(diméthoxy-2,2 éthyl)-4 dioxo-5,6 tétrahydro-1,4,5,6 triazine-1,2,4 yl-3] thiométhyl-3 oxo-8 oxyde-5 thia-5 aza-1 bicyclo[4.2.0] octène-2 dans 80 cm3 de dichlorométhane en présence de 20 cm3 d'une solution demi-saturée de bicarbonate de sodium. On décante, lave la phase organique par 2 fois 20 cm3 d'une solution demi-saturée de chlorure de sodium, sèche sur sulfate de sodium et filtre. Au filtrat, on ajoute 2 g d'acide méthoxyimino-2 (tritylamino-2 thiazolyl-4)-2 acétique, isomère syn et 25 mg de diméthylamino-4 pyridine. On refroidit à +5°C et ajoute en 15 mn une solution de 1,02 g de N,N'-dicyclohexylcarbodiimide dans 10 cm3 de dichlorométhane, on laisse remonter la température à +20°C et agite pendant 3 heures. On dilue le mélange par 100 cm3 d'acétate d'éthyle, filtre, lave successivement par 2 fois 10 cm3 d'acide chlorhydrique 0,05 N, 20 cm3 d'une solution saturée de bicarbonate de sodium et 20 cm3 d'une solution demi-saturée de chlorure de sodium, sèche sur sulfate de sodium, filtre et concentre à sec à 20°C sous 20 mm de mercure (2,7 kPa). On fixe le résidu sur 20 g de gel de silice Merck (0,06-02) et chromatographie sur une colonne de 80 g de gel de silice (diamètre de la colonne : 2,6 cm, hauteur : 39 cm). On élue par 250 cm3 d'un mélange cyclohexane-acétate d'éthyle 50-50 (en volumes) 250 cm3 d'un mélange 40-60, 500 cm3 d'un mélange 30-70, 500 cm3 d'un mélange 20-80 et 2 1 d'acétate d'éthyle en recueillant des fractions de 60 cm3. On concentre à sec les fractions 33 à 55 à 20°C sous 20 mm de mercure (2,7 kPa) et recueille 1,52 g de benzhydryloxycarbonyl-2 [(diméthoxy-2,2 éthyl)-4 dioxo-5,6 tétrahydro-1,4,5,6 triazine-1,2,4 yl-3] thiométhyl-3 [méthoxyimino-2 (tritylamino-2 thiazolyl-4)-2 acétamido]-7 oxo-8 oxyde-5 thia-5 aza-1 bicyclo[4.2.0] octène-2, isomère syn sous la forme d'une meringue jaune.

Spectre infra-rouge (KBr), bandes caractéristiques ($cm^{-1}$) : 1805, 1720, 1695, 1590, 1525, 1495, 1450, 1050, 750, 700.

Spectre RMN du proton (350 MHz, DMSO $d_6$, δ en ppm, J en Hz) : 3,20 à 3,40 (mf, $-OCH_3$ + DOH) ; 3,76 et 3,9 à 4 (d, J = 18, 1H et mt, $-CH_2S(O)-$) ; 3,85 (s, 3H, $=NOCH_3$) ; 3,9 (mt, 2H, $>NCH_2-$) ; 3,9 à 4 et 4,26 (mt et d, J = 14, 1H, $-SCH_2-$) ; 4,55 (t, J = 5, 1H, $-\underline{CH}(OCH_3)_2$) ; 4,99 (d, J = 4, 1H, H en 6) ; 5,83 (dd, J = 4 et 8, 1H, H en 7) ; 6,8 (s, 1H, H du thiazole) ; 7,01 (s, 1H, $-COOCH<$) ; 8,76 (s, 1H, $-\underline{NH}C(C_6H_5)_3$) ; 8,96 (d, J = 8, -CONH-) ; 12,53 (s, 1H, =NNHCO- ou =NN=ÇOH).

On traite à -5°C pendant 15 minutes une solution de 1,48 g de benzhydryloxycarbonyl-2 [(diméthoxy-2,2 éthyl)-4 dioxo-5,6 tétrahydro-1,4,5,6 triazine-1,2,4 yl-3] thiométhyl-3 [méthoxyimino-2 (tritylamino-2 thiazolyl-4)-2 acétamido]-7 oxo-8 oxyde-5 thia-5 aza-1 bicyclo[4.2.0] octène-2, isomère syn dans un mélange de 10 cm3 de dichlorométhane et 0,56 cm3 de N,N-diméthylacétamide par 0,25 cm3 de trichlorure de phosphore. On dilue le mélange par 100 cm3 d'acétate d'éthyle, lave par 50 cm3 d'une solution demi-saturée de bicarbonate de sodium et 2 fois 50 cm3 d'une solution demi-saturée de chlorure de sodium, sèche sur sulfate de sodium, filtre et concentre à sec à 20°C sous 20 mm de mercure (2,7 kPa). On fixe le résidu sur 10 g de sel de silice Merck (0,06-0,2) et chromatographie sur une colonne de 20 g de gel de silice (diamètre de la colonne : 2 cm ; hauteur 20 cm). On élue par 125 cm3 d'un mélange cyclohexane-acétate d'éthyle 50-50 (en volumes), 125 cm3 d'un mélange 25-75 (en volumes) et 300 cm3 d'acétate d'éthyle en recueillant des fractions de 50 cm3. Les fractions 4 à 8 sont concentrées à sec à 20°C sous 20 mm de mercure (2,7 kPa), on recueille 0,85 g de benzhydryloxycarbonyl-2 [(diméthoxy-2,2 éthyl)-4 dioxo-5,6 tétrahydro-1,4,5,6 triazine-1,2,4 yl-3] thiométhyl-3 [méthoxyimino-2 (tritylamino-2 thiazolyl-4)-2 acétamido]-7 oxo-8 thia-5 aza-1 bicyclo [4.2.0] octène-2, isomère syn sous la forme d'une meringue de couleur crème.

Spectre infra-rouge (KBr), bandes caractéristiques ($cm^{-1}$) : 1790, 1720, 1695, 1590, 1525, 1500, 1450, 1080, 1050, 750, 700.

BAD ORIGINAL

Spectre de RMN du proton (350 MHz, DMSO $d_6$, δ en ppm, J en Hz) : 3,30 (mf, $-OCH_3$ + DOH) ; 3,62 et 3,78 (2d, J = 17,5, 2H, $-CH_2S-$) ; 3,87 (s, 3H, $=NOCH_3$) ; 3,95 (mt, $>NCH_2-$) ; 3,96 et 4,15 (2d, J = 12, 2H, $-CH_2S-$ exo) ; 4,58 (t, J = 5, 1H, $-C\underline{H}(OCH_3)_2$) ; 5,20 (d, J = 5, 1H, H en 6) ; 5,79 (dd, J = 5 et 9, 1H, H en 7) ; 6,75 (s, 1H, H du thiazole) ; 6,99 (s, 1H, -COOCH<) ; 8,83 (s, 1H, $-N\underline{H}C(C_6H_5)_3$) ; 9,6 (d, J = 9, 1H, -CONH-) ; 12,53 (s, 1H, =NNHCO- ou =NN=COH).

On agite à 50°C pendant 30 minutes une solution de 0,61 g de benzhydryloxycarbonyl-2 [(diméthoxy-2,2 éthyl)-4 dioxo-5,6 tétrahydro-1,4,5,6 triazine-1,2,4 yl-3] thiométhyl-3 [méthoxyimino-2 (tritylamino-2 thiazolyl-4)-2 acétamido]-7 oxo-8 thia-5 aza-1 bicyclo[4.2.0] octène-2, isomère syn dans 15 cm3 d'acide formique pur. On ajoute ensuite 1,5 cm3 d'eau, agite à nouveau pendant 10 mn à 50°C et concentre à sec à 30°C sous 0,05 mm de mercure. On reprend le résidu dans 3 fois 50 cm3 d'acétone en évaporant à chaque fois à 20°C sous 20 mm de mercure (2,7 kPa), traite le solide obtenu par 50 cm3 d'acétone à reflux sous agitation, filtre à chaud, lave par 30 cm3 d'éther et sèche sous 0,05 mm de mercure. On recueille 0,26 g d'[(amino-2 thiazolyl-4)-2 méthoxyimino-2 acétamido]-7 carboxy-2 (dioxo-5,6 formylméthyl-4 tétrahydro-1,4,5,6 triazine-1,2,4 yl-3) thiométhyl-3 oxo-8 thia-5 aza-1 bicyclo[4.2.0] octène-2, isomère syn, sous la forme d'une poudre de couleur crème.

Spectre infra-rouge (KBr), bandes caractéristiques ($cm^{-1}$) : 3300, 3200, 3100, 2200, 1780, 1715, 1680, 1610, 1530, 1040.

Spectre de RMN du proton (350 MHz, $CF_3COOD$, δ en ppm, J en Hz) : On observe les principaux signaux suivants : 4,80 (s, 3H, $=NOCH_3$) ; 5,19 (s large, 2H, $>NCH_2-$) ; 5,34 (s, 1H, H en 6) ; 6,04 (s, 1H, H en 7) ; 9,73 (s, 1H, -CHO).

L'amino-7 benzhydryloxycarbonyl-2 [(diméthoxy-2,2 éthyl)-4 dioxo-5,6 tétrahydro-1,4,5,6 triazine-1,2,4 yl-3] thiométhyl-3 oxo-8 oxyde-5 thia-5 aza-1 bicyclo[4.2.0] octène-2 peut être obtenu de la manière suivante :

On agite à 20°C pendant 3 heures un mélange de 4,92 g de benzhydryloxycarbonyl-2 [(diméthoxy-2,2 éthyl)-4 dioxo-5,6 tétrahydro-1,4,

5,6 triazine-1,2,4 yl-3] thiométhyl-3 formamido-7 oxo-8 oxyde-5 thia-5 aza-1 bicyclo[4.2.0] octène-2, 72 cm3 de méthanol et 17,5 cm3 d'une solution 4 N d'acide chlorhydrique anhydre dans l'éther éthylique. On filtre la suspension, lave le solide à l'éther et sèche sous 0,05 mm de mercure pendant 12 heures. On recueille 3,05 g de chlorhydrate d'amino-7 benzhydryloxycarbonyl-2 [(diméthoxy-2,2 éthyl)-4 dioxo-5,6 tétrahydro-1,4,5,6 triazine-1,2,4 yl-3] thiométhyl-3 oxo-8 oxyde-5 thia-5 aza-1 bicyclo[4.2.0] octène-2 sous la forme d'une poudre beige.

Spectre infra-rouge (KBr), bandes caractéristiques ($cm^{-1}$) : 3300, 3200, 1800, 1715, 1590, 1495, 1455, 1080, 1050, 740, 700.

Spectre de RMN du proton (350 MHz, DMSO $d_6$, $\delta$ en ppm, J en Hz) : 3,25 et 3,27 (2s, 6H, $-OCH_3$) ; 3,50 (mf ; $-NH_3^+$ + DOH) ; 3,92 (mt, 2H, $>NCH_2-$) ; 3,93 et 4,26 (2d, J = 13, 2H, $-CH_2S-$) ; 4,01 et 4,11 (2d, J = 18, 2H, $-CH_2S(O)-$) ; 4,55 (t, J = 5, 1H, $-\underline{CH}(OCH_3)_2$) ; 5,11 (d, J = 4, 1H, H en 6) ; 5,35 (d, J = 4, 1H, H en 7) ; 7,05 (s, 1H, -COOCH<) ; 12,59 (s, 1H, =NNHCO- ou =NN=ÇOH).

Le benzhydryloxycarbonyl-2 [(diméthoxy-2,2 éthyl)-4 dioxo-5,6 tétrahydro-1,4,5,6 triazine-1,2,4 yl-3] thiométhyl-3 formamido-7 oxo-8 oxyde-5 thia-5 aza-1 bicyclo[4.2.0] octène-2 peut être obtenu de la manière suivante :

On agite à 20°C pendant 10 heures sous azote, un mélange de 5,03 g de benzhydryloxycarbonyl-2 bromométhyl-3 formamido-7 oxo-8 oxyde-5 thia-5 aza-1 bicyclo[4.2.0] octène-2, 20 cm3 de diméthylformamide et 3,81 g du sel de sodium de la (diméthoxy-2,2 éthyl)-4 dioxo-5,6 thioxo-3 perhydrotriazine-1,2,4. On verse la solution dans 200 cm3 d'eau, filtre, lave le solide par 4 fois 25 cm3 d'eau et sèche à l'air. On obtient 5,31 g de benzhydryloxycarbonyl-2 [(diméthoxy-2,2 éthyl)-4 dioxo-5,6 tétrahydro-1,4,5,6 triazine-1,2,4 yl-3] thiométhyl-3 formamido-7 oxo-8 oxyde-5 thia-5 aza-1 bicyclo[4.2.0] octène-2 sous la forme d'une poudre beige.

Spectre infra-rouge (KBr), bandes caractéristiques ($cm^{-1}$) : 3400, 3260, 1790, 1715, 1690, 1590, 1495, 1455, 1080, 1055, 740, 700.

Spectre de RMN du proton (350 MHz, DMSO $d_6$, $\delta$ en ppm, J en Hz) : 3,2 à 3,5 (mf, $-OCH_3$ + DOH) ; 3,78 et 4,03 (2d, J = 18, 2H, $-CH_2S(O)-$) ;

3,90 (mt, 2H, $>NCH_2-$) ; 3,93 et 4,28 (2d, J = 13, 2H, $-CH_2S-$) ; 4,55 (t, J = 5, 1H, $-C\underline{H}(OCH_3)_2$) ; 4,98 (d, J = 4, 1H, H en 6) ; 6,03 (dd, J = 4 et 9, 1H, H en 7) ; 7,02 (s, 1H, $-COOCH<$) ; 8,19 (s, 1H, -CHO) ; 8,44 (d, J = 9, 1H, -CONH-) ; 12,54 (s, 1H, =NNHCO- ou =NN=COH)

La (diméthoxy-2,2 éthyl)-4 dioxo-5,6 thioxo-3 perhydrotriazine-1,2,4 peut être préparée de la manière suivante :

On prépare une solution de méthylate de sodium par dissolution de 4,15 g de sodium dans 140 cm3 de méthanol, ajoute 32,3 g de (diméthoxy-2,2 éthyl)-4 thiosemicarbazide et ajoute 26,3 g d'oxalate d'éthyle. On porte à reflux sous agitation pendant 4 heures et laisse refroidir. Après une nuit, la suspension obtenue est filtrée et le précipité lavé par 3 fois 25 cm3 d'éther. Le solide est mis en solution dans 40 cm3 d'eau et, après refroidissement vers 4°C, la solution est acidifiée à pH 3 par de l'acide chlorhydrique 4N et laissée à 4°C pendant 30 minutes. Après filtration et séchage, on recueille 12 g de (diméthoxy-2,2 éthyl)-4 dioxo-5,6 thioxo-3 perhydrotriazine-1,2,4 sous la forme d'un solide blanc.

F. inst.(Kofler) = 172°C (déc.).

Spectre infra-rouge (KBr), bandes caractéristiques en $cm^{-1}$ : 3280, 3250, 1695, 1380, 1130, 1050

Spectre de RMN du proton ( 80 MHz, DMSO $d_6$, δ en ppm, J en Hz) : 3,30 (s, 6H, $-CH(OC\underline{H}_3)_2$); 4,38 (d, J = 5,5, 2H, $>NCH_2-$) ; 4,94 (t, J = 5,5, 1H, $-C\underline{H}(OCH_3)_2$).

La (diméthoxy-2,2 éthyl)-4 thiosemicarbazide peut être préparée de la manière suivante :

A une solution de 14,35 g d'hydrate d'hydrazine dans 40 cm3 d'éthanol on ajoute en 1 heure, sous agitation à une température comprise entre 5 et 9°C, 37,7 g d'isothiocyanate de diméthoxy-2,2 éthyle. Après 12 heures à 4°C, le mélange est concentré à sec à 20°C sous pression réduite (20 mm de mercure ; 2,7 kPa). Le sirop jaune obtenu cristallise après amorçage. Le solide est dissous à chaud dans 50 cm3 de méthanol, on filtre et dilue par 200 cm3 d'éther diéthylique. Après une dizaine d'heures à 4°C, on filtre et recueille 32,3 g de (diméthoxy-2,2 éthyl)-4 thiosemicarbazide sous la forme d'un solide blanc.

F. inst. (Kofler) = 69°C.

La présente invention concerne également les médicaments qui contiennent comme produit actif au moins un produit de formule générale (I) à l'état pur (sous forme libre ou sous forme de sel) ou sous forme d'une composition en association avec un ou plusieurs adjuvants pharmaceutiquement acceptables. Ces médicaments peuvent être utilisés par voie orale, parentérale ou rectale.

Comme compositions solides pour administration orale peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également préparées sous forme de compositions solides stériles qui seront dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou des glycérides semi-synthétiques.

BAD ORIGINAL

En thérapeutique humaine, les médicaments selon la présente invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 1 et 10 g par jour de produit actif par voie orale, intramusculaire ou intraveineuse pour un adulte.

L'exemple suivant, donné à titre non limitatif, illustre une composition selon la présente invention.

EXEMPLE -

On prépare 100 cm3 d'une solution aqueuse isotonique contenant 1,44 g de bicarbonate de sodium et comme produit actif 10 g d'(amino-2 thiazolyl-4)-2 méthoxyimino-2 acétamido]-7 carboxy-2 [(dioxo-5,6 formylméthyl-4 tétrahydro-1,4,5,6 triazine-1,2,4 yl-3) thiométhyl-3 oxo-8 thia-5 aza-1 bicyclo[4.2.0] octène-2, isomère syn.

Après filtration sur filtre bactériologique, on répartit aseptiquement cette solution en ampoules (à raison de 10 cm3 par ampoule), on lyophilise et on scelle les ampoules.

Chaque ampoule contient l'équivalent de 1 g du produit actif sous forme de son sel de sodium.

BAD ORIGINAL

REVENDICATIONS

1 - Un nouveau dérivé de la céphalosporine caractérisé en ce qu'il répond à la formule générale :

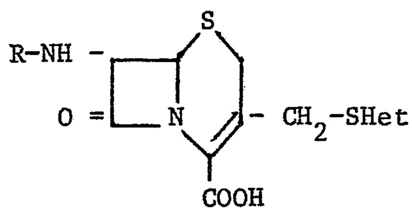

dans laquelle

le symbole R représente un radical (amino-2 thiazolyl-4)-2 acétyle, (amino-2 thiazolyl-4)-2 méthoxyimino-2 acétyle, (amino-2 thiazolyl-4)-2 carboxyalcoyloxyimino-2 acétyle [dont la partie carboxyalcoyle peut être représentée par le radical de formule générale :

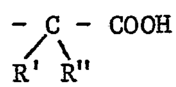

dans laquelle R' et R" qui sont identiques ou différents représentent des atomes d'hydrogène ou des radicaux alcoyle (contenant 1 à 4 atomes de carbone), ou forment ensemble un radical alcoylène contenant 2 ou 3 atomes de carbone], un radical N-(dioxo-2,3 éthyl-4 pipérazinyl-carbonyl) Dα-p.hydroxyphénylglycyle ou N-(hydroxy-4 méthyl-6 pyridyl-3) carbonyl Dα-p.hydroxyphénylglycyle, et

le symbole Het représente :

1° un radical dioxo-5,6 tétrahydro-1,4,5,6 triazine-1,2,4 yle-3, substitué en position -1 ou -4 ou un radical dioxo-5,6 tétrahydro-1,2,5,6 triazine-1,2,4 yle-3 substitué en position -2 par un radical formylalcoyle, ou bien

2° un radical alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1 par un radical formylalcoyle, étant entendu que (sauf

mention spéciale) les radicaux et portions alcoyle cités ci-dessus contiennent 1 ou 2 atomes de carbone, ainsi que ses sels métalliques ou ses sels d'addition avec les bases azotées et lorsqu'ils existent ses sels d'addition avec les acides.

2 - Un nouveau dérivé de la céphalosporine selon la revendication 1, caractérisé en ce que le symbole Het représente un radical dioxo-5,6 formylalcoyl-4 tétrahydro-1,4,5,6 triazine-1,2,4 yle-3.

3 - Un procédé pour la préparation d'un produit selon la revendication 1 caractérisé en ce que l'on acyle une amino-7 céphalosporine de formule générale :

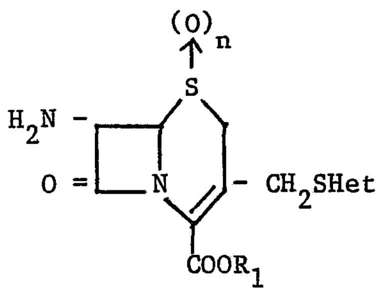

(dans laquelle Het est défini selon la revendication 1, $R_1$ représente un atome d'hydrogène ou un radical protecteur d'acide facilement éliminable et n représente 0 ou 1) au moyen d'un acide représenté par la formule générale :

$$R - OH$$

dans laquelle R est défini comme dans la revendication 1 ou d'un dérivé réactif de cet acide, puis on réduit le sulfoxyde lorsque n = 1, élimine les radicaux protecteurs et transforme éventuellement le produit obtenu en un sel métallique, un sel d'addition avec une base azotée, ou lorsqu'il existe en un sel d'addition avec un acide.

4 - Procédé selon la revendication 3 caractérisé en ce que l'on utilise une amino-7 céphalosporine dans laquelle $R_1$ est un radical protecteur choisi parmi méthoxyméthyle, t.butyle, benzhydryle, benzyle, nitrobenzyle ou p.méthoxybenzyle.

5 - Un procédé pour la préparation d'un produit selon la revendication 1 caractérisé en ce que l'on fait agir un thiol (ou un sel alcalin ou alcalino-terreux d'un thiol) de formule générale :

Het - SH

[dans laquelle Het est défini selon la revendication 1 étant entendu que le substituant formylalcoyle est protégé à l'état d'acétal de formule générale :

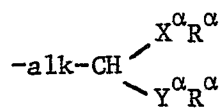

dans laquelle alk est un radical alcoylène contenant 1 ou 2 atomes de carbone, $X^{\alpha}$ et $Y^{\alpha}$ sont identiques et représentent des atomes d'oxygène ou de soufre et $R^{\alpha}$ représente un radical alcoyle, ou bien $X^{\alpha}$ et $Y^{\alpha}$ sont identiques ou différents et représentent des atomes d'oxygène ou de soufre et les radicaux $R^{\alpha}$ forment ensemble un radical alcoylène contenant 2 ou 3 atomes de carbone], sur un dérivé de la céphalosporine de formule générale :

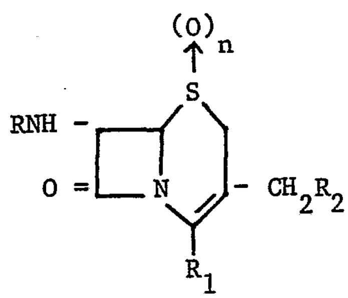

dans laquelle R est défini comme dans la revendication 1, $R_2$ représente un atome d'halogène choisi parmi le chlore, le brome et l'iode, $R_1$ et n sont définis comme dans la revendication 3 ou bien $R_2$ représente un radical acétoxy, $R_1$ représente un atome d'hydrogène et n est égal à 0, puis on réduit le sulfoxyde obtenu lorsque n = 1, élimine les radicaux protecteurs et transforme éventuellement le produit obtenu en un sel métallique, en un sel d'addition avec une base azotée, ou lorsqu'il existe en un sel d'addition avec un acide.

6 - Un procédé pour la préparation d'un produit selon la revendication 1 caractérisé en ce que l'on fait agir un thioloester représenté par la formule générale :

R-S-Het

dans laquelle R et Het sont définis selon la revendication 1 [étant entendu que lorsque R contient un substituant amino, celui-ci est protégé et que le substituant formylalcoyle de Het est protégé à l'état d'acétal tel que défini dans la revendication 5] sur une amino-7 céphalosporine de formule générale :

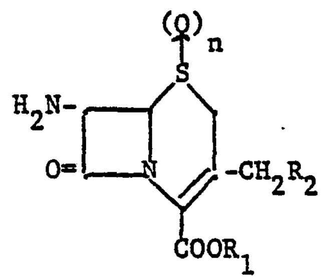

dans laquelle $R_1$, $R_2$ et n sont définis comme dans la revendication 5, puis réduit le sulfoxyde obtenu lorsque n = 1, élimine les groupements protecteurs et transforme éventuellement le produit obtenu en un sel métallique, en un sel d'addition avec une base azotée ou lorsqu'il existe en un sel d'addition avec un acide.

7 - Un procédé pour la préparation d'un produit selon la revendication 1 pour lequel R représente un radical (amino-2 thiazolyl-4)-2 acétyle, (amino-2 thiazolyl-4)-2 méthoxyimino-2 acétyle ou (amino-2 thiazolyl-4)-2 carboxyalcoyloxyimino-2 acétyle [dont la partie carboxyalcoyle est définie comme dans la revendication 1], caractérisé en ce que l'on fait agir une thiourée de formule générale :

$R_4NHCSNH_2$

dans laquelle $R_4$ est un atome d'hydrogène ou un radical protecteur d'amino, sur un produit de formule générale :

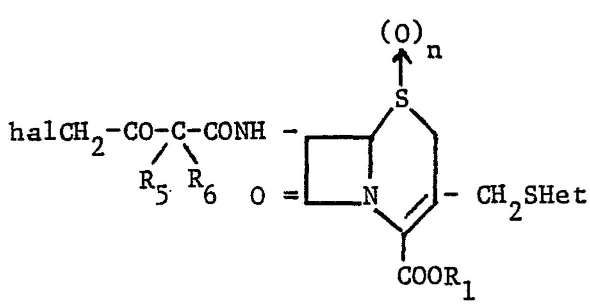

dans laquelle Het est défini comme dans la revendication 1, $R_1$ et n sont définis comme dans la revendication 3, $R_5$ et $R_6$ soit représentent simultanément des atomes d'hydrogène, soit forment ensemble un radical méthoxyimino ou carboxyalcoyloxyimino libre ou protégé et hal représente un atome de chlore ou de brome, puis on réduit le sulfoxyde obtenu lorsque n = 1, élimine éventuellement les radicaux protecteurs et transforme éventuellement le produit obtenu en un sel métallique, en un sel d'addition avec une base azotée ou en un sel d'addition avec un acide.

8 - Un procédé selon la revendication 7 caractérisé en ce que l'on utilise une thiourée dans laquelle $R_4$ est un radical protecteur d'amine choisi parmi formyle, t.butoxycarbonyle, p.méthoxybenzyloxycarbonyle, p.nitrobenzyloxycarbonyle, trityle ou trichloro-2,2,2 éthoxycarbonyle.

9 - Composition pharmaceutique caractérisée en ce qu'elle comprend au moins un produit selon la revendication 1 en association avec un ou plusieurs diluants ou adjuvants compatibles et pharmaceutiquement acceptables.

Revendication spéciale Autriche

## REVENDICATION

Procédé de préparation d'un nouveau dérivé de la céphalosporine de formule générale :

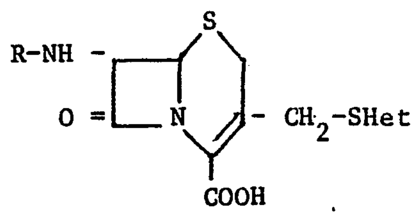

dans laquelle
le symbole R représente un radical (amino-2 thiazolyl-4)-2 acétyle, (amino-2 thiazolyl-4)-2 méthoxyimino-2 acétyle, (amino-2 thiazolyl-4)-2 carboxyalcoyloxyimino-2 acétyle [dont la partie carboxyalcoyle peut être représentée par le radical de formule générale :

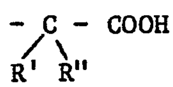

dans laquelle R' et R" qui sont identiques ou différents représentent des atomes d'hydrogène ou des radicaux alcoyle (contenant 1 à 4 atomes de carbone), ou forment ensemble un radical alcoylène contenant 2 ou 3 atomes de carbone], un radical N-(dioxo-2,3 éthyl-4 pipérazinyl-carbonyl) Dα-p.hydroxyphénylglycyle ou N-(hydroxy-4 méthyl-6 pyridyl-3) carbonyl Dα-p.hydroxyphénylglycyle, et
le symbole Het représente :
1° un radical dioxo-5,6 tétrahydro-1,4,5,6 triazine-1,2,4 yle-3, substitué en position -1 ou -4 ou un radical dioxo-5,6 tétrahydro-1,2,5,6 triazine-1,2,4 yle-3 substitué en position -2 par un radical formylalcoyle, ou bien
2° un radical alcoyl-1 dioxo-5,6 tétrahydro-1,4,5,6 triazine-1,2,4 yle-3 substitué en position -4, alcoyl-1 dioxo-5,6 tétrahydro-1,2,5,6 triazine-1,2,4 yle-3 substitué en position -2, alcoyl-2 dioxo-5,6 tétrahydro-1,2,5,6 triazine-1,2,4 yle-3 substitué en position -1 ou alcoyl-4 dioxo-5,6 tétrahydro-1,4,5,6 triazine-1,2,4 yle-3 substitué en position -1 par un radical formylalcoyle, étant entendu que (sauf

mention spéciale) les radicaux et portions alcoyle cités ci-dessus contiennent 1 ou 2 atomes de carbone, ainsi que ses sels métalliques ou ses sels d'addition avec les bases azotées et lorsqu'ils existent ses sels d'addition avec les acides, caractérisé en ce que

A - on acyle une amino-7 céphalosporine de formule générale :

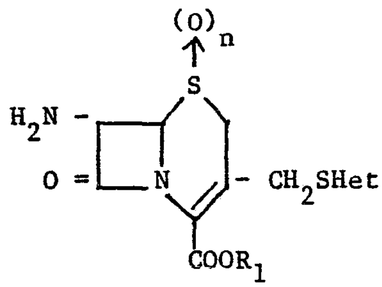

(dans laquelle Het est défini comme ci-dessus, $R_1$ représente un atome un atome d'hydrogène ou un radical protecteur d'acide facilement éliminable et n représente 0 ou 1) au moyen d'un acide représenté par la formule générale :

R - OH

dans laquelle R est défini comme ci-dessus ou d'un dérivé réactif de cet acide, puis on réduit le sulfoxyde lorsque n = 1, élimine les radicaux protecteurs et transforme éventuellement le produit obtenu en un sel métallique, un sel d'addition avec une base azotée, ou lorsqu'il existe en un sel d'addition avec un acide, ou

B - on fait agir un thiol (ou un sel alcalin ou alcalino-terreux d'un thiol) de formule générale :

Het - SH

[dans laquelle Het est défini comme ci-dessus, étant entendu que le substituant formylalcoyle est protégé à l'état d'acétal de formule générale :

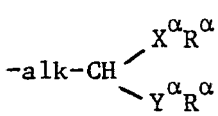

dans laquelle alk est un radical alcoylène contenant 1 ou 2 atomes de carbone, $X^{\alpha}$ et $Y^{\alpha}$ sont identiques et représentent des atomes d'oxygène ou de soufre et $R^{\alpha}$ représente un radical alcoyle, ou bien $X^{\alpha}$ et $Y^{\alpha}$

sont identiques ou différents et représentent des atomes d'oxygène ou de soufre et les radicaux $R^{\alpha}$ forment ensemble un radical alcoylène contenant 2 ou 3 atomes de carbone], sur un dérivé de la céphalosporine de formule générale :

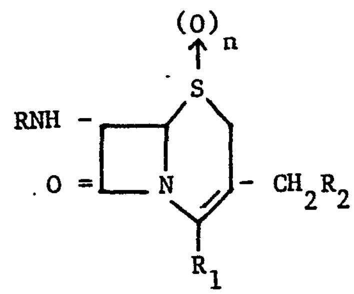

dans laquelle R est défini comme ci-dessus, $R_2$ représente un atome d'halogène choisi parmi le chlore, le brome et l'iode, $R_1$ et n sont définis comme en A- ou bien $R_2$ représente un radical acétoxy, $R_1$ représente un atome d'hydrogène et n est égal à 0, puis on réduit le sulfoxyde obtenu lorsque n = 1, élimine les radicaux protecteurs et transforme éventuellement le produit obtenu en un sel métallique, en un sel d'addition avec une base azotée, ou lorsqu'il existe en un sel d'addition avec un acide, ou

C - on fait agir un thioloester représenté par la formule générale :

R-S-Het

dans laquelle R et Het sont définis comme ci-dessus [étant entendu que lorsque R contient un substituant amino, celui-ci est protégé et que le substituant formylalcoyle de Het est protégé à l'état d'acétal tel que défini ci-dessus] sur une amino-7 céphalosporine de formule générale :

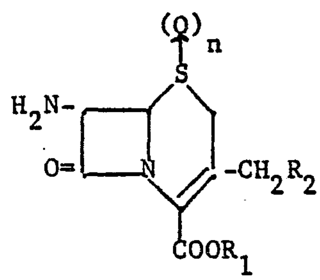

dans laquelle $R_1$, $R_2$ et n sont définis comme en B-, puis on réduit le sulfoxyde obtenu lorsque n = 1, élimine les groupements protecteurs et transforme éventuellement le produit obtenu en un sel métallique, en un sel d'addition avec une base azotée ou lorsqu'il existe en un sel d'addition avec un acide, ou

D - pour la préparation d'un produit dans la formule duquel R représente un radical (amino-2 thiazolyl-4)-2 acétyle, (amino-2 thiazolyl-4)-2 méthoxyimino-2 acétyle, (amino-2 thiazolyl-4)-2 carboxyalcoyloxyimino-2 acétyle [dont la partie carboxyalcoyle est définie comme ci-dessus] on fait agir une thiourée de formule générale :

$$R_4NHCSNH_2$$

dans laquelle $R_4$ est un atome d'hydrogène ou un radical protecteur d'amino, sur un produit de formule générale :

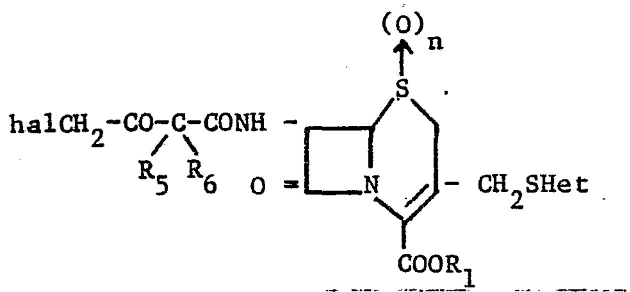

dans laquelle Het est défini comme ci-dessus, $R_1$ et n sont définis comme en A-, $R_5$ et $R_6$ soit représentent simultanément des atomes d'hydrogène, soit forment ensemble un radical méthoxyimino ou carboxyalcoyloxyimino libre ou protégé et hal représente un atome de chlore ou de brome, puis on réduit le sulfoxyde obtenu lorsque n = 1, élimine éventuellement les radicaux protecteurs et transforme éventuellement le produit obtenu en un sel métallique, en un sel d'addition avec une base azotée ou en un sel d'addition avec un acide.

0053539

Office européen des brevets

# RAPPORT DE RECHERCHE EUROPEENNE

Numéro de la demande

EP 81 40 1826

## DOCUMENTS CONSIDERES COMME PERTINENTS

| Catégorie | Citation du document avec indication, en cas de besoin, des parties pertinentes | Revendication concernée |
|---|---|---|
| A | EP - A - 0 002 605 (ELI LILLY)<br>* Pages 36-38; revendications * | 1-9 |
| | -- | |
| A | FR - A - 2 280 381 (ELI LILLY)<br>* Pages 43-47; revendications * | 1-9 |
| | -- | |
| A | FR - A - 2 275 215 (HOFFMANN-LA ROCHE)<br>* Document en entier * | 1-9 |
| | -- | |
| A | FR - A - 2 427 337 (HOFFMANN-LA ROCHE)<br>* Pages 22-25; revendications * | 1-9 |
| | ---- | |

**CLASSEMENT DE LA DEMANDE (Int. Cl. 3)**

C 07 D 501/36
A 61 K 31/545//
C 07 D 253/06
C 07 C 159/00

**DOMAINES TECHNIQUES RECHERCHES (Int. Cl. 3)**

C 07 D 501/00

**CATEGORIE DES DOCUMENTS CITES**

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention
E: document de brevet antérieur, mais publié à la date de dépôt ou après cette date
D: cité dans la demande
L: cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 04-03-1982 | LUYTEN |

OEB Form 1503.1 06.78